# EUROPEAN PATENT SPECIFICATION

(11) **EP 3 513 185 B1**
(45) Date of publication and mention of the grant of the patent: **19.11.2025**
(21) Application number: 17771561.2
(22) Date of filing: 18.09.2017
(51) Int. Cl.: G01N 33/487, C12M 1/34, C12Q 1/02

(54) **BIOMASS MONITORING PROCESS**
BIOMASSEÜBERWACHUNGSVERFAHREN
PROCÉDÉ DE SURVEILLANCE DE BIOMASSE

(30) Priority: 16.09.2016 GB 201615814; 07.07.2017 GB 201711001
(43) Date of publication of application: 24.07.2019
(73) Proprietor: Aber Instruments Ltd, Dyfed SY23 3AH (GB)
(72) Inventor: JONES, Steffan Keri, Aberystwyth SY23 3AH (GB); YEOMANS, Paul Geraint, Aberystwyth SY23 3AH (GB); TWIDDY, Peter John Cameron, Aberystwyth SY23 3AH (GB); PRYCE, Tim William, Aberystwyth SY23 3AH (GB)
(74) Representative: Adamson Jones
(86) International application number: PCT/GB2017/052768
(87) International publication number: WO 2018/051135

(56) References cited:
- EP-A1- 1 046 905
- EP-A2- 2 381 249
- WO-A1-94/07123
- DE-A1- 19 611 931
- US-A- 4 160 205
- US-A- 4 893 935
- MATTHEW; GAVIN BRUCE: "THE USE OF CAPACITANCE MEASUREMENT IN FERMENTATION MONITORING", 1 September 1999 (1999-09-01), pages 1 - 215, XP093134538, Retrieved from the Internet <URL:https://discovery.ucl.ac.uk/id/eprint/10098635/1/The_use_of_capicitance_measure.pdf> [retrieved on 20240223]

## Description

Capacitance measurement techniques are known for measuring the capacitance (or specific capacitance or dielectric constant) of liquids and suspensions, such as biological cells in ionic aqueous solutions.

Monitoring systems incorporating such measurement capability are beneficial for measuring concentration of live cells. In particular in the brewing industry, the concentration of live yeast can be measured with an on-line capacitance probe. A radio frequency applied from the electrodes of the probe causes ions in the suspending medium (for example wort or green beer) and the cytoplasm of the yeast to move towards the two respective oppositely charged electrodes. As the plasma membrane is non-conducting a build up of charge results in the cells and are said to be polarised with the yeast cells acting as tiny capacitors within the medium. Non-viable cells or cells with a damaged membrane do not interfere with the signal. Thus, a build up of charge cannot occur as the ions can freely move across the membrane and so the cells do not become polarised. The measured capacitance is directly proportional to the amount of viable yeast within a sample over a wide concentration range. Such technology can also be utilised for measuring biomass in the field of biotechnology, for example, in controlling cell culture processes.

A variety of different probe arrangements have been utilised to measure biomass. Generally speaking, biomass sensing probes can be divided into two groups, namely disposable or single-use probes and reusable probes.

One significant property of a biomass sensing probe which determines whether it is reusable or single-use is its resistance to sterilisation. Once a biomass probe has been used to obtain biomass measurements in a first medium, it cannot then be used to obtain such measurements in a second medium until it has been sterilised. Sterilisation is necessary to ensure that trace amounts of residual cell-containing medium deposited on the probe after use with the first medium do not influence biomass readings obtained when the probe is used subsequently in the second medium, as well as to prevent contamination of the second medium with the residual first medium.

The problem of inter-medium contamination is particularly acute for biotechnological applications as contamination of the products of such applications can pose serious health risks to the users of those products. Accordingly, in accordance with regulatory requirements, biomass sensing probes (and other components of culturing systems) must be sterilised once used if they are to be used again in subsequent biomass sensing operations.

As those skilled in the art will be aware, there are a number of techniques for sterilising biomass sensing probes including dry heat sterilisation (e.g. in an autoclave), steam sterilisation and gamma irradiation. It will be recognised that such techniques in which probes are exposed to harsh conditions can damage them, resulting in the probes becoming deficient. Probe damage caused by sterilisation is a particularly acute concern in the field of biomass sensing given that the capacitance signals detected by the probes in use are relatively weak and thus even a minor reduction in probe sensitivity can render the probe inutile. The issue of damage to electrodes caused by sterilisation is considered in UK Patent No. GB2177801.

A number of reusable probes, i.e. probes which are resistant to damage caused by sterilisation have been commercialised. For example Aber Instruments Limited have commercialised a series of reusable probes for use in both the brewing and biotechnological industries, for example pinned electrode, flush electrode and annular electrode probes sold under the Futura^{®} brand. Futura^{®} brand probes have been demonstrated as being usable following 50 (and for more recently developed probes, 100) sterilisations by steam, dry heat or autoclave.

Additional reusable biomass probes are disclosed in UK Patent No. 2479783 and UK Patent No. 2507283.

Thus, while the production of reusable biomass sensing probes is possible, their construction is complex in order for them to be resistant to sterilisation. Further, they must be formed from high-grade materials and thus the cost of reusable biomass sensing probes is relatively high. While investment in reusable biomass sensing probes may be justified in certain applications, in other cases, a lower cost option may be preferable.

Single-use probes are generally less costly than reusable probes. As a result of them not having to be resistant to sterilisation, their construction is typically less complex and the materials from which they are produced are less costly.

Additionally, the end user does not need to invest in sterilisation apparatus, the implementation and operation of which on an industrial scale can add significantly to operating cost. The obvious disadvantage of single-use probes is their limited applicability; as the name suggests, they can be used only once. Accordingly, in applications where repeated biomass readings are required, a substantial number of single-use probes will be required which will add to cost, as well as to environmental burden as after use; the probes will typically be disposed of.

Ph.D Thesis from Matthew Gavin Bruce entitled: "THE USE OF CAPACITANCE MEASUREMENT IN FERMENTATION MONITORING", 1 September 1999 (1999-09-01), pages 1-215, XP09313453: https://discovery.ucl.ac.uk/id/ eprint/10098635 discloses the use of capacitance probes in biomass monitoring systems.

Thus, while conventional biomass sensing probes provide a number of options to the biotechnological and brewing industries, a need remains for a reusable biomass probe which has a less complex construction than conventional reusable biomass sensing probes and / or which is less costly and / or complex to produce than conventional biomass sensing probes.

According to a first aspect of the present invention, there is provided a process according to independent claim 1 for obtaining a plurality of biomass measurements from one or more biological medium, the process comprising:
i) providing a biomass sensing probe comprising a body and at least one electrode provided on a first region of the body;
ii) contacting the at least one electrode and the first region of the body with a biological medium and obtaining a first biomass reading indicative of the number of live cells within the biological medium;
iii) removing the at least one electrode and the first region of the body from the biological medium;
iv) sterilising the at least one electrode and the first region of the body; and
v) contacting the at least one electrode and the first region of the body with a biological medium and obtaining a second biomass reading indicative of the number of live cells within the biological medium;
wherein the first region of the body is formed from liquid crystal polymer, phenolic polymer, nylon, polyethylene, polypropylene, polystyrene, polyvinylchloride, acrylonitrile butadiene styrene, acetal resins, sulphone, polysulphone, polyamide, polyphenylene sulphide, polyetheretherketone, polyethylene terephthalate, polyetherketone, polyoxymethylene, polyphthalamide, polyetherketoneketone, thermoplastic polyimide, polyacrylate, polytetrafluoroethylene, polymethylmethacrylate or mixtures thereof.

As is apparent, the process of the present invention permits the re-use of a biomass sensing probe formed at least partly from plastic. Probes for use in the process of the present invention are relatively straightforward to manufacture, less complex in construction than conventional re-usable probes, and are also less costly than conventional re-usable probes. The ability of biomass sensing probes formed at least partly from plastic to be resistant to sterilisation such that they can be reused to take subsequent biomass readings is surprising given the level of sensitivity needed for biomass sensor probes.

The provision of a probe having a body formed at least partially of plastics material which is capable of resisting damage caused by sterilisation is advantageous as it permits not only the electrode to be sterilised, but also at least a portion of the body to also to be sterilised (despite being formed of plastic).

This is a significant advance over arrangements of the prior art, such as that disclosed in GB2177801 in which only the electrodes are subjected to sterilisation, meaning that other components employed in that arrangement are not sterilised (and are not capable of being repeatedly sterilised) thus posing the risk of contamination.

The process of the present invention includes a single inter-use sterilisation step, i.e. a sterilisation step between the first and second biomass readings. However, in embodiments of the invention, the probe may be sterilised and viably reused a plurality of times, despite being formed of plastic.

Thus, in embodiments of the invention, the process further comprises vi) removing the at least one electrode and the first region of the body from the biological medium, vii-a) sterilising the at least one electrode and the first region of the body, vii-b) contacting the at least one electrode and the first region of the body with a biological medium and obtaining a further biomass reading indicative of the number of live cells within the biological medium, and vii-c) removing the electrode and the first region of the body from the biological medium.

In embodiments of the invention, the biological media from which the first biomass reading, the second biomass reading, and further biomass readings (if performed) may be the same or different media. For example, the first biomass reading may be taken from a first biological medium and the second biomass reading may be taken from a second biological medium different from the first biological medium. Further biomass readings may be taken from biological media which are the same as or different to the first and / or second biological media.

In embodiments of the invention, steps vii-a) to vii-c) are repeated 3 or more, 8 or more, 13 or more, or 18 or more times, i.e. such that in total, the probe is used to take 5 or more, 10 or more, 15 or more or 20 or more biomass readings.

Those skilled in the art will recognise that the process will only be of use if the reading is within acceptable limits of accuracy. Thus, for the avoidance of doubt, where reference is made to obtaining a biomass reading as part of the process of the present invention, this biomass reading must be viable. To determine the viability of a biomass reading of a probe, the probe (once properly calibrated) when used with a standard conductivity solution such as potassium chloride (e.g. provided as a Standard Reference Material by NIST under reference number SRM2202) or potassium fluoride (e.g. provided as a Standard Reference Material by NIST under reference number SRM2203) must provide a conductivity reading which varies from the reference value of the standard conductivity solution by about 5% or less, about 3% or less, about 2% or less, or by about 1% or less. Advantageously, the probes for use in the process of the present invention are capable of meeting this requirement following multiple sterilisations.

In processes of the present invention, the sterilisation step carried out in step iv) and / or step vii-a) may be any type of sterilisation known to those skilled in the art which can deliver the required level of sterility for the desired application. In embodiments of the invention, the sterilisation step carried out in step iv) and / or step vii-a) may be heat, chemical (e.g. caustic, bleach and / or alcohol), steam and / or gamma sterilisation.

As mentioned above, the steps iv) and iiv-a) require that the at least one electrode and the first region of the body is subjected to sterilisation. The purpose of this step is to ensure that the part of the probe body which comes into contact with biological medium (the first region, at least) is sterilised. However, for convenience, or to minimise the risk of contamination, in embodiments of the invention, step iv) and / or step iiv-a) may involve sterilisation of the entire body and the electrode/s of the probe. Additionally or alternatively, the probe may be sterilised in the same sterilisation operation as other components to be used in the processes of the present invention. For example, in embodiments in which the biomass reading is taken from medium in a bioreactor, the bioreactor and the probe may be sterilised in the same operation.

The probe for use in the process of the present invention may be of any style provided that it permits biomass readings to be taken from biological media. Those skilled in the art will be familiar with elongate biomass probes and disc-style probes. Either type of construction can be employed in the process of the present invention.

Thus, where the probe employed in the process of the present invention has a elongate construction, the body of the probe is generally elongate, terminating in a tip at its distal end. The body may have any shape, for example it may be circular, square or polygonal in cross section and / or be hollow or solid.

In the elongate embodiment, the body may have a diameter ranging from about about 5mm to about 15mm. The length of the body ranges from about 100mm, to about 250mm.

In such embodiments, the at least one electrode may extend along a portion of the outer surface of the body and / or may be positioned at the tip of the body. The electrode/s may extend longitudinally along a part of or all of the body.

Additionally or alternatively, the electrode/s may have an annular configuration, extending around a part of the body, or be trapezoidally arranged on the probe, e.g. at its end. Examples of how electrodes may be configured in elongate probes are disclosed in UK Patent No. 2507283.

The at least one electrode may be connected to one or more conducting means (e.g. wires, tracks or the like) to carry the biomass reading signal to a position distant from the electrode/s. Such conducting means may be positioned within the body of the probe.

The elongate probe useful in the processes of the present invention may be formed via a range of manufacturing methods. In embodiments of the invention, the body of the probe is monolithic and may be formed in one or more moulding (e.g. injection moulding) or extrusion step/s.

Alternatively, the body of a elongate probe may be modular. For example, it may comprise a tip part and an elongate part. The elongate part may be formed in one or more moulding (e.g. injection moulding) or extrusion step/s. In such an arrangement, the tip may comprise the at least one electrode and optionally be substantially solid. The elongate part may be hollow to permit conducting means (e.g. wires, tracks or the like) to pass through its interior which conducting means can then be connected to the electrode/s in the tip. Alternatively, the elongate part may be solid and have conducting means moulded into its interior, which conducting means can then be connected to the at least one electrode in the tip. Once any conducting means within the elongate portion of the body have been connected to the at least one electrode in the tip portion, the elongate portion and the tip portion can be coupled together, e.g. adhesively, to provide a watertight seal in order to prevent the ingress of biological medium into the interior of the body.

Additionally or alternatively, the elongate portion (having the tip and / or electrode integrally formed therein or coupled thereto) may be coupled to connection means and / or coupling means, and / or be formed integrally with connection means and / or coupling means. In arrangements in which the elongate portion is coupled to the connection means and / or coupling means, this may be achieved using any approach known to those skilled in the art, for example using adhesive to provide a seal preventing the ingress of contaminants or biological medium into the interior of the probe.

Examples of the adhesive which may be employed to couple the elongate member to the tip, coupling means, connection means and / or any other component include cyanoacrylates, methacrylates, polyolefins (e.g. hot melt polyolefins), polyurethanes, epoxy resins, for example one part epoxy resins and / or two part epoxy resins.

In embodiments in which components of the probe are adhered to other components, the probe component may be treated to improve adhesion. For example, the probe component may be cleaned, e.g. with a solvent cleaner.

Additionally or alternatively, the surface of the probe component may be primed, e.g. with a chemical primer and / or the application of plasma energy.

In an alternative embodiment, the probe may be a disc-shaped probe. In such an embodiment, the body of the probe is typically generally cylindrical, having flat, circular surfaces at its upper and lower ends. However, in embodiments of the invention, the body may generally prismic, having a different shape in cross section, e.g. square or polygonal.

In such embodiments, the at least one electrode is positioned on one of the surfaces, while connection means are positioned at the other surface, the connection means being connected to the at least one electrode via conducting means (e.g. wires, tracks or the like) which pass through the body.

The disc-shaped probe may be provided with an annular flange extending around some or all of the circumference / perimeter of the body.

Regardless of the configuration of the probe, it may contain any number of electrodes. For example, the probe may contain 1, 2, 3, 4, 5, 6, 7, 8 or more than 8 electrodes. In embodiments of the invention, the probe may contain 2 to 6 or 2 to 4 electrodes.

In arrangements in accordance with the invention where the probe has an elongate construction, the first region and at least one electrode may be located at an end of the probe.

Those skilled in the art will be familiar with materials from which the at least one electrode may be formed or plated. As examples, the electrode/s may be formed of or plated with gold, stainless steel, iridium, platinum (e.g. platinum black) and any other material with low or controllable electrode polarisation properties.

The probe for use in the process of the present invention may comprise conducting means (e.g. tracks which may be formed in printed circuit board (PCB) or in the body of the probe, wires, or the like) to carry biomass signals to a location remote from the at least one electrode. The conducting means may be formed from any electrically conductive material, for example copper.

As explained above, body of the probe is formed of plastic. The plastic preferably is medically approved, has high mechanical strength, is resistant to chemical degradation and has high temperature stability.

In order to be reusable, the type of plastic must be selected to be able to withstand sterilisation conditions. In embodiments of the invention, the body is formed of plastic which is resistant to steam, heat and / or gamma irradiation sterilisation. For example, the plastic may have a melting point of at least about 130°C in order to be resistant to autoclave sterilsation, or at least about 170°C in order to be resistant to steam and heat sterilisation. In embodiments of the invention, the plastic from which the body is formed has a melting point of at least about 110°C, at least about 115°C, at least about 120°C, at least about 130°C, at least about 150°, at least about 170°C, at least about 190°C, or at least about 200°C.

Additionally or alternatively, the plastic from which the body is formed is gamma-irradiatable. In embodiments of the invention, the plastic from which the body is formed makes no observable changes when exposed to gamma irradiation at a dose of about 10kGy, about 20kGy, about 30kGy, about 40kGy, or about 50kGy.

In embodiments of the invention, the material from which the first region of the probe body (and optionally some or all of the remainder of the probe body) is formed is an injection mouldable plastic. In such embodiments, the body of the probe is formed by injection moulding.

Examples of polymeric materials which may be used to form first region of the body of the probe which is to be used in the processes of the present invention include liquid crystal polymers such as aromatic polyesters (e.g. polymers sold under the trade name Vectra^{®} and Zenite^{®} by Celanese), phenolic, nylon (e.g. high temperature nylon), polyethylene (e.g. HDPE, LDPE), polypropylene (e.g. polymers sold by Ineos) polystyrene, polyvinylchloride, acrylonitrile butadiene styrene, acetal resins (e.g. polymers sold under the trade name Delrin^{®} by Du Pont), sulphone (e.g. polymers sold under the trade names Radel^{®} PPSU, Veradel^{®} PESU, Udel^{®} PSU, Acudel^{®} modified PPSU by Solvay) polysulphone, polyamide, polyphenylene sulphide, polyetheretherketone, polyethylene terephthalate, polyetherketone, polyoxymethylene, polyphthalamide, polyetherketoneketone, thermoplastic polyimide (e.g. polyetherimide or polyamideimide), polyacrylate, polytetrafluoroethylene, polymethylmethacrylate or mixtures thereof.

In embodiments of the invention, both the first region of the body of the probe and some or all of the remainder of the probe body are formed from the polymeric materials recited in the preceding paragraph.

In embodiments, the first region of the body of the probe used in the process of the present invention is formed (either partially or completely) of semi-crystalline and / or amorphous polymers. Additionally, or alternatively, both the first region of the body of the probe and some or all of the remainder of the probe body are formed (either partially or completely) of semi-crystalline and / or amorphous polymers.

The biological media (first, second and / or further) which are contacted with the electrode/s of the probe in the process of the present invention may be contained within a bioreactor. The bioreactor may be a reusable bioreactor, for example a fermenter formed from stainless steel, glass, plastic and has a capacity ranging from 10mL to about 950mL. Alternatively, the bioreactor may be a single use bioreactor, for example a bag type bioreactor (e.g. marketed by Applikon, Broadley James, Cellexus, Eppendorf, Finesse, GE Lifesciences, Infors, Pall, or Sartorius Stedim). Whether reusable or single use, the bioreactor may be provided with a port or opening via which the probe can be inserted and optionally connected, in order to contact the biological medium with the at least one electrode on the probe.

The process of the invention may advantageously be conducted with any type of biological media. Biotechnological and brewing applications have been mentioned above, but these are merely illustrative. The biological medium (e.g. first, second and / or further biological media) may be liquid and contain a plurality of cells. The cells may be human, animal (mammal or other), bacterial, plant, stem, fungal (e.g. yeast) or other.

In embodiments, the probe may be calibrated during the process of the present invention. Such a calibration may take place at any stage in the process, but preferably takes place prior to step ii). The probe for use in the process of the present invention may be calibrated using any technique known to those skilled in the art. For example, the probe may be placed in one or more solutions of standard conductivity (for example potassium chloride (e.g. provided as a Standard Reference Material by NIST under reference number SRM2202) or potassium fluoride (e.g. provided as a Standard Reference Material by NIST under reference number SRM2203)) and a conductivity measurement taken. In the event that a correction is needed for the observed conductivity measurement to equate to that of the standard solution/s, then this correction is applied to future readings obtained from the probe.

In embodiments of the invention, the probe for use in the process of the present invention may be equipped with data storage means, for example a microchip. The data storage means may store information such as calibration correction values, serial and / or part numbers, temperature values or other information.

In embodiments of the invention, biomass signal processing means may be employed in order to take a biomass reading. Those skilled in the art will be familiar with biomass signal processing means, for example the Futura^{®} range of systems (Aber Instruments Limited).

The biomass signal processing means are preferably in communication with the probe to enable the transmission of biomass signals from the probe to the biomass signal processing means. For example, the probe may be coupled directly to the biomass signal processing means, either permanently or removably.

Alternatively, the connection between the probe and the biomass signal processing means may be a cable. The cable may be coupled to the probe either permanently or removably.

In a further embodiment, the connection between the probe and the biomass signal processing means may be wireless, e.g. via WLAN, bluetooth, RFID, NFC, or the like.

In embodiments in which the probe is removably coupled to other components, e.g. to a cable or directly to the biomass signal processing means, the probe may be provided with coupling means to enable the probe to be connected to other components. In embodiments of the invention, the coupling means comprises a plug / socket part (e.g. which is complementary to the component to which the probe is to be connected), a slide connector, a push-pull connector (e.g. as commercialised by Redel), a flexible catch and / or a screwed connector.

In embodiments the probe may be provided pre-sterilised, i.e. so that it can be used without the need for a prior sterilisation step. In such embodiments, the probe may be provided in sterile packaging and thus the process comprises the step of removing the probe from its sterile packaging, e.g. in a sterile environment such as a special air flow hood.

Additionally or alternatively, the process may comprise a preliminary sterilisation step, optionally carried out prior to step ii). Again, this sterilisation step may be a steam, heat and / or gamma irradiation sterilisation step.

As will be recognised from the disclosure herein, a significant advance of the process of the present invention is the use of a biomass sensor probe having a body formed of plastic.

Thus, according to a further aspect of the present invention, there is provided a reusable biomass sensor probe for use in taking a plurality of biomass readings from one or more biological medium, wherein the probe is resistant to sterilisation.

The present invention will now be described in the examples which follow.

### Example 1 - Reusable Plastic Probe

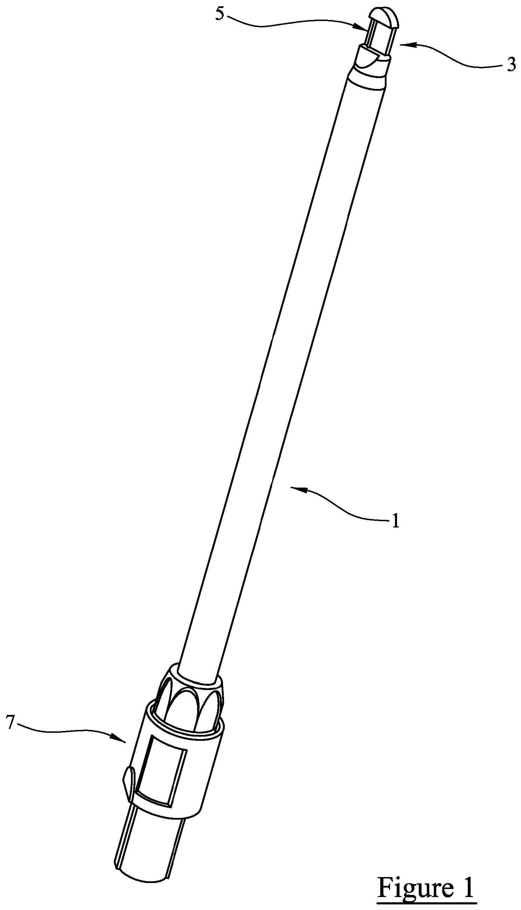

An example of a reusable probe having a plastic body is illustrated in Figure 1. The body of the probe is formed of a semi-crystalline liquid crystal polymer. As can be seen the probe (1) has an elongate construction. It is modular in construction and comprises an injection moulded elongate portion and a tip (3). The tip (3) has four electrodes (5) formed therein which are trapezoidally arranged (only two electrodes (5) are visible in Figure 1). The tip (3) is adhesively coupled to the elongate body using an epoxy resin. At the other end of the probe (1), a push-pull Redel connector (7) is provided, to conveniently enable the probe to be connected to signal processing means (not shown). The connector (7) is adhesively coupled to the elongate body using an epoxy resin.

### Example 2 - Resistance to Autoclave Sterilisation

Two probes (identified with the references PT26 and PT30) having the construction detailed in Example 1 were subjected to a series of tests to demonstrate its ability to be re-used multiple time, i.e. to take accurate biomass measurements despite being repeatedly sterilised.

Probes PT26 and PT30 were subjected to a series of autoclave cycles at 121°C for one hour, during each autoclave cycle, the distal ends of the probes were submerged in saline solution. Following each autoclave cycle the probes were allowed to dry for one hour. The degradation of the probe / electrodes was assessed in terms of tests 1 and 2 below. The performance of the probes against the saline standard was assessed in terms of test 3 below:
1) leakage - the electrical resistance between electrodes of the probe was measured. A measured value of ≥32MΩ is considered a pass, <32MΩ and ≥1MΩ where a leakage is recorded is considered a pass. <1MΩ is considered to be a fail.
2) corrosion level - the potential difference between electrodes of the probe was determined and if a [reading / variation] of no greater than 50mV was observed, this was considered to be a pass,
3) capacitance - the capacitance of the standard saline solution was measured using the probes.

Regarding the results for tests 1) and 2), these are set out in Table 1 below.

**Table 1 - Results of Leakage Resistance and Corrosion Tests**

| Autoclave Cycles | PT26 | PT30 |
|---|---|---|
| 1 | ✔ | ✔ |
| 2 | ✔ | ✔ |
| 3 | ✔ | ✔ |
| 4 | ✔ | ✔ |
| 5 | ✔ | ✔ |
| 6 | ✔ | ✔ |
| 7 | ✔ | ✔ |
| 8 | Fail | ✔ |
| 9 | ✔ | ✔ |
| 10 | ✔ | ✔ |
| 11 | ✔ | ✔ |
| 12 | Fail | ✔ |
| 13 | ✔ | ✔ |
| 14 | ✔ | Fail |
| 15 | ✔ | ✔ |
| 16 | ✔ | ✔ |
| 17 | ✔ | ✔ |
| 18 | ✔ | ✔ |
| 19 | ✔ | ✔ |
| 20 | ✔ | ✔ |
| 21 | ✔ | ✔ |
| 22 | ✔ | ✔ |
| 23 | ✔ | ✔ |
| 24 | ✔ | ✔ |
| 25 | Fail | ✔ |
| 26 | Fail | ✔ |
| 27 | ✔ | ✔ |
| 28 | ✔ | ✔ |
| 29 | | ✔ |
| 30 | | ✔ |

As is apparent from the data shown in Table 1, the probes of Example 1 were both capable of being sterilised seven times with no reduction in leakage resistance or build up of a corrosion signal observed. While there were fails following the 8th and 12th autoclave cycles for PT26 and following the 14th autoclave cycle for PT30, these were relatively minor.

Further, as can be seen, this drop in performance was reversed in subsequent tests which strongly suggests that the fails which were observed were not permanent, structural issues with the probes but more likely issues with the testing methodology, potentially on the grounds that there was not sufficient time between cycles to allow the probes to dry fully which affected the measurements.

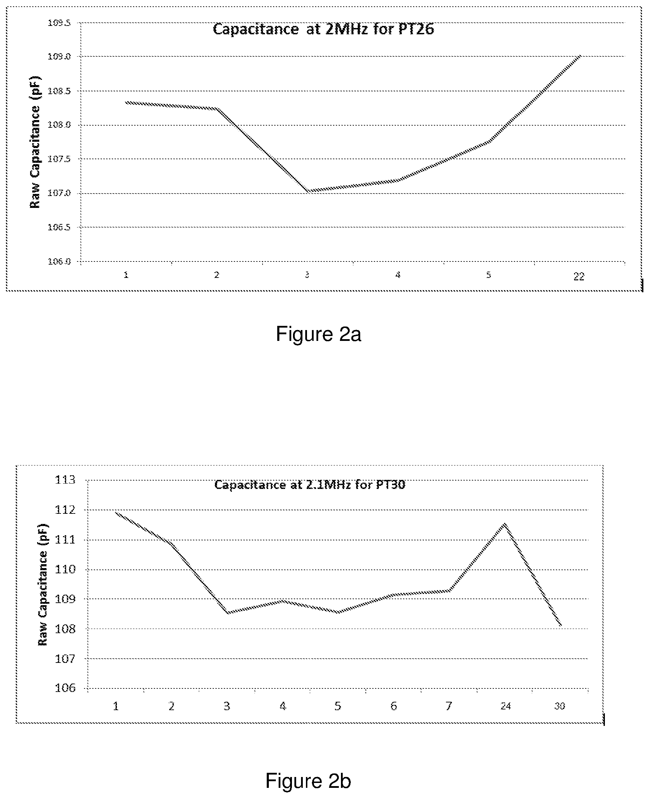

The results of test 3) (capacitance) are shown in Figures 2a and 2b. As can be seen, the extent to which repeated sterilisation impacted the capacitance measurements made by the probes was negligible. This data demonstrates that, unlike conventional plastic probes, the probes of the present invention can be sterilised by autoclave and reused in excess of 20 times to take reliable capacitance measurements.

## Claims

1. A process for obtaining a plurality of biomass measurements from one or more biological medium, the biological medium being contained within a bioreactor, and said bioreactor having a capacity of from about 10mL to about 950mL, the process comprising:
i) providing a biomass sensing probe comprising a body and at least one electrode provided on a first region of the body, wherein the probe has an elongate construction and the body of the probe has a diameter of about 5mm to about 15mm, and a length of about 100mm to about 250mm;
ii) contacting the at least one electrode and a first region of the body with a biological medium and obtaining a first capacitance based biomass reading indicative of the number of live cells within the biological medium;
iii) removing the at least one electrode and the first region of the body from the biological medium;
iv) sterilising the at least one electrode and the first region of the body; and
v) contacting the at least one electrode and the first region of the body with a biological medium and obtaining a second capacitance based biomass reading indicative of the number of live cells within the biological medium;
wherein the first region of the body is formed from liquid crystal polymer, phenolic polymer, nylon, polyethylene, polypropylene, polystyrene, polyvinylchloride, acrylonitrile butadiene styrene, acetal resins, sulphone, polysulphone, polyamide, polyphenylene sulphide, polyetheretherketone, polyethylene terephthalate, polyetherketone, polyoxymethylene, polyphthalamide, polyetherketoneketone, thermoplastic polyimide, polyacrylate, polytetrafluoroethylene, polymethylmethacrylate or mixtures thereof.

2. The process of Claim 1, further comprising the steps of:
vi) removing the at least one electrode and the first region of the body from the biological medium,
vii-a) sterilising the at least one electrode and the first region of the body,
vii-b) contacting the at least one electrode and the first region of the body with a biological medium and obtaining a further biomass reading indicative of the number of live cells within the biological medium, and
vii-c) removing the electrode and the first region of the body from the biological medium,
wherein optionally steps vii-a) to vii-c) are repeated 3 or more times, or 13 or more times.

3. The process of Claim 1 or 2, wherein the sterilisation step carried out in step iv) and / or step vii-a) is a heat and / or steam sterilisation.

4. The process of any one of Claims 1 to 3, wherein in step iv) and / or step iiv-a) the entire body and the electrode/s of the probe are sterilised.

5. The process of any one of Claims 1 to 4, wherein the body of the probe is circular in cross section.

6. The process of any one of Claims 1 to 5, wherein the probe comprises a tip, and the tip comprises one or more electrodes, or optionally 2 to 6 electrodes.

7. The process of any one of Claims 1 to 6, wherein the body of the probe is injection moulded.

8. The process of any one of Claims 1 to 7, wherein the first region of the body is formed of a semi-crystalline polymeric material or a liquid crystal polymer.

9. The process of any one of Claims 1 to 8, further comprising the step of calibrating the probe, wherein optionally the step of calibrating the probe precedes step ii) of the process.

10. The process of any one of Claims 1 to 9, further comprising a preliminary sterilisation step, which precedes step ii), wherein optionally the preliminary sterilisation step is a gamma sterilisation.

11. The process of any one of Claims 1 to 10, wherein the biological medium from which the first biomass reading is obtained in step ii) is a first biological medium and the biological medium from which the second biomass reading is obtained in step v) is a second biological medium, the second biological medium being different from the first biological medium.

12. The process of Claim 11, wherein the biological medium from which the further biomass reading is obtained in step vii-b) is a further biological medium which is different from the first and / or second biological medium.

## Patentansprüche

1. Verfahren zum Erhalten einer Vielzahl von Biomassemessungen aus einem oder mehreren biologischen Medien, wobei das biologische Medium in einem Bioreaktor enthalten ist und der Bioreaktor eine Kapazität von etwa 10 ml bis etwa 950 ml aufweist, wobei das Verfahren Folgendes umfasst:
i) Bereitstellen einer Biomasse-Sensorsonde, die einen Körper und mindestens eine Elektrode umfasst, die an einem ersten Bereich des Körpers vorgesehen ist, wobei die Sonde eine längliche Konstruktion aufweist und der Körper der Sonde einen Durchmesser von etwa 5 mm bis etwa 15 mm und eine Länge von etwa 100 mm bis etwa 250 mm aufweist;
ii) Kontaktieren der mindestens einen Elektrode und eines ersten Bereichs des Körpers mit einem biologischen Medium und Erhalten eines ersten kapazitätsbasierten Biomassemesswerts, der bezeichnend für die Anzahl der lebenden Zellen innerhalb des biologischen Mediums ist;
iii) Entfernen der mindestens einen Elektrode und des ersten Bereichs des Körpers aus dem biologischen Medium;
iv) Sterilisieren der mindestens einen Elektrode und des ersten Bereichs des Körpers; und
v) Kontaktieren der mindestens einen Elektrode und des ersten Bereichs des Körpers mit einem biologischen Medium und Erhalten eines zweiten kapazitätsbasierten Biomassemesswerts, der bezeichnend für die Anzahl der lebenden Zellen innerhalb des biologischen Mediums ist;
wobei der erste Bereich des Körpers aus Flüssigkristallpolymer, Phenolpolymer, Nylon, Polyethylen, Polypropylen, Polystyrol, Polyvinylchlorid, Acrylnitrilbutadienstyrol, Acetalharzen, Sulfon, Polysulfon, Polyamid, Polyphenylensulfid, Polyetheretherketon, Polyethylenterephthalat, Polyetherketon, Polyoxymethylen, Polyphthalamid, Polyetherketonketon, thermoplastischem Polyimid, Polyacrylat, Polytetrafluorethylen, Polymethylmethacrylat oder Mischungen davon gebildet ist.

2. Verfahren nach Anspruch 1, ferner umfassend die folgenden Schritte:
vi) Entfernen der mindestens einen Elektrode und des ersten Bereichs des Körpers aus dem biologischen Medium,
vii-a) Sterilisieren der mindestens einen Elektrode und des ersten Bereichs des Körpers,
vii-b) Kontaktieren der mindestens einen Elektrode und des ersten Bereichs des Körpers mit einem biologischen Medium und Erhalten eines weiteren Biomassemesswerts, der bezeichnend für die Anzahl der lebenden Zellen innerhalb des biologischen Mediums ist, und
vii-c) Entfernen der Elektrode und des ersten Bereichs des Körpers aus dem biologischen Medium,
wobei optional die Schritte vii-a) bis vii-c) 3 oder mehr Mal oder 13 oder mehr Mal wiederholt werden.

3. Verfahren nach Anspruch 1 oder 2, wobei der in Schritt iv) und/oder Schritt vii-a) durchgeführte Sterilisationsschritt eine Wärme- und/oder Dampfsterilisation ist.

4. Verfahren nach einem der Ansprüche 1 bis 3, wobei in Schritt iv) und/oder Schritt iiv-a) der gesamte Körper und die Elektrode(n) der Sonde sterilisiert werden.

5. Verfahren nach einem der Ansprüche 1 bis 4, wobei der Körper der Sonde im Querschnitt kreisförmig ist.

6. Verfahren nach einem der Ansprüche 1 bis 5, wobei die Sonde eine Spitze umfasst und die Spitze eine oder mehrere Elektroden oder optional 2 bis 6 Elektroden umfasst.

7. Verfahren nach einem der Ansprüche 1 bis 6, wobei der Körper der Sonde spritzgegossen ist.

8. Verfahren nach einem der Ansprüche 1 bis 7, wobei der erste Bereich des Körpers aus einem halbkristallinen Polymermaterial oder einem Flüssigkristallpolymer gebildet ist.

9. Verfahren nach einem der Ansprüche 1 bis 8, ferner umfassend den Schritt des Kalibrierens der Sonde, wobei optional der Schritt des Kalibrierens der Sonde dem Schritt ii) des Verfahrens vorausgeht.

10. Verfahren nach einem der Ansprüche 1 bis 9, ferner umfassend einen Vorsterilisationsschritt, der dem Schritt ii) vorausgeht, wobei optional der Vorsterilisationsschritt eine Gammasterilisation ist.

11. Verfahren nach einem der Ansprüche 1 bis 10, wobei das biologische Medium, aus dem der erste Biomassemesswert in Schritt ii) erhalten wird, ein erstes biologisches Medium ist und das biologische Medium, aus dem der zweite Biomassemesswert in Schritt v) erhalten wird, ein zweites biologisches Medium ist, wobei sich das zweite biologische Medium von dem ersten biologischen Medium unterscheidet.

12. Verfahren nach Anspruch 11, wobei das biologische Medium, aus dem der weitere Biomassemesswert in Schritt vii-b) erhalten wird, ein weiteres biologisches Medium ist, das sich von dem ersten und/oder zweiten biologischen Medium unterscheidet.

## Revendications

1. Procédé permettant d'obtenir une pluralité de mesures de biomasse à partir d'un ou plusieurs milieux biologiques, le milieu biologique étant contenu dans un bioréacteur, et ledit bioréacteur ayant une capacité d'environ 10 ml à environ 950 ml, le procédé comprenant :
i) la fourniture d'une sonde de détection de biomasse comprenant un corps et au moins une électrode située sur une première région du corps, ladite sonde présentant une construction allongée et ledit corps de ladite sonde présentant un diamètre d'environ 5 mm à environ 15 mm et une longueur d'environ 100 mm à environ 250 mm ;
ii) la mise en contact de l'au moins une électrode et d'une première région du corps avec un milieu biologique et l'obtention d'une première lecture de biomasse basée sur la capacité, indiquant le nombre de cellules vivantes dans le milieu biologique ;
iii) le retrait de l'au moins une électrode et de la première région du corps du milieu biologique ;
iv) la stérilisation de l'au moins une électrode et de la première région du corps ; et
v) la mise en contact de l'au moins une électrode et de la première région du corps avec un milieu biologique et l'obtention d'une seconde lecture de biomasse basée sur la capacité, indiquant le nombre de cellules vivantes dans le milieu biologique ;
ladite première région du corps étant formée de polymère à cristaux liquides, de polymère phénolique, de nylon, de polyéthylène, de polypropylène, de polystyrène, de polychlorure de vinyle, d'acrylonitrile butadiène styrène, de résines d'acétal, de sulfone, de polysulfone, de polyamide, de polysulfure de phénylène, de polyétheréthercétone, de polyéthylène téréphtalate, de polyéthercétone, de polyoxyméthylène, de polyphtalamide, de polyéthercétonecétone, de polyimide thermoplastique, de polyacrylate, de polytétrafluoroéthylène, de polyméthylméthacrylate ou de mélanges de ceux-ci.

2. Procédé selon la revendication 1, comprenant en outre les étapes suivantes :
vi) le retrait de l'au moins une électrode et de la première région du corps du milieu biologique,
vii-a) la stérilisation de l'au moins une électrode et de la première région du corps,
vii-b) la mise en contact de l'au moins une électrode et de la première région du corps avec un milieu biologique et l'obtention d'une nouvelle lecture de biomasse indiquant le nombre de cellules vivantes dans le milieu biologique, et
vii-c) le retrait de l'électrode et de la première région du corps du milieu biologique,
lesdites étapes vii-a) à vii-c) étant éventuellement répétées 3 fois ou plus ou 13 fois ou plus.

3. Procédé selon la revendication 1 ou 2, ladite étape de stérilisation effectuée à l'étape iv) et/ou l'étape vii-a) étant une stérilisation à la chaleur et/ou à la vapeur.

4. Procédé selon l'une quelconque des revendications 1 à 3, à ladite étape iv) et/ou à ladite étape vii-a), ledit corps entier et ladite ou lesdites électrodes de la sonde étant stérilisés.

5. Procédé selon l'une quelconque des revendications 1 à 4, ledit corps de ladite sonde présentant une section transversale circulaire.

6. Procédé selon l'une quelconque des revendications 1 à 5, ladite sonde comprenant une pointe et ladite pointe comprenant une ou plusieurs électrodes ou éventuellement 2 à 6 électrodes.

7. Procédé selon l'une quelconque des revendications 1 à 6, ledit corps de ladite sonde étant moulé par injection.

8. Procédé selon l'une quelconque des revendications 1 à 7, ladite première région dudit corps étant formée d'un matériau polymère semi-cristallin ou d'un polymère à cristaux liquides.

9. Procédé selon l'une quelconque des revendications 1 à 8, comprenant en outre l'étape d'étalonnage de la sonde, ladite étape d'étalonnage de la sonde précédant éventuellement l'étape ii) du procédé.

10. Procédé selon l'une quelconque des revendications 1 à 9, comprenant en outre une étape de stérilisation préliminaire, qui précède l'étape ii), ladite étape de stérilisation préliminaire étant éventuellement une stérilisation gamma.

11. Procédé selon l'une quelconque des revendications 1 à 10, ledit milieu biologique à partir duquel la première lecture de biomasse est obtenue à l'étape ii) étant un premier milieu biologique et ledit milieu biologique à partir duquel la seconde lecture de biomasse est obtenue à l'étape v) étant un second milieu biologique, le second milieu biologique étant différent du premier milieu biologique.

12. Procédé selon la revendication 11, ledit milieu biologique à partir duquel la nouvelle lecture de biomasse est obtenue à l'étape vii-b) étant un autre milieu biologique qui est différent des premier et/ou second milieux biologiques.
